# EUROPEAN PATENT APPLICATION

(11) **EP 4 134 974 A1**
(43) Date of publication of application: **15.02.2023**
(21) Application number: 21191091.4
(22) Date of filing: 12.08.2021
(51) Int. Cl.: G16H 40/20

(54) **DYNAMIC CARE ASSISTANCE MECHANISM**

(71) Applicant: Koninklijke Philips N.V., 5656 AG Eindhoven (NL)
(72) Inventor: VASIST, Santosh Narasimhaswamy, 5656 AE Eindhoven (NL); BERA, Deep, 5656 AE Eindhoven (NL); VAN LIESHOUT, Ron Martinus Laurentius, 5656 AE Eindhoven (NL); BULUT, Murtaza, 5656 AE Eindhoven (NL); SARTOR, Francesco, 5656 AE Eindhoven (NL)
(74) Representative: Philips Intellectual Property & Standards

(57) **Abstract**

A mechanism for controlling how assistance is provided to a caregiver or clinician. Information about a workload and amount of information available to the caregiver is obtained. This information is processed to determine a recommended level of assistance that should be provided to the caregiver. The operation of an assistance tool, which may be run by a processing system, is modified based upon the recommended level of assistance.

## Description

### FIELD OF THE INVENTION

The present invention relates to the field of providing assistance to a caregiver, and in particular, to a caregiver that interacts with a user interface providing information about a subject.

### BACKGROUND OF THE INVENTION

The nature of medical care demands a high level of skill, experience and specialization from caregivers. There is an increasing concern about the shortage of available caregivers in society, together with a trend towards younger or more inexperienced caregivers taking on more responsibilities, e.g. becoming responsible for more subjects (i.e. patients). These two factors, combined with a busy clinical area, can overload the caregivers reducing their efficiency.

Subject monitors have advanced significantly in recent times and make available, via their user interfaces, a large amount of information to the caregiver for interpretation and analysis. The information is usually provided at a display, in the form of numbers and curves and are displayed over multiple tabs/windows on the display. By way of example, the Philips IntelliVue MX800 subject monitor can have up to 20 windows/tabs simultaneously open. It is also noted that some parameters may have similar ranges or potential values, e.g. blood pressure, oxygen saturation and pulse rate which have a similar range of values, can all take a value of 95.

The large amount and numeric overlap of clinical data available for review by a caregiver increase a risk that certain information will be overlooked or missed.

The present disclosure recognizes a desire to provide mechanisms that aid a caregiver in identifying information of a subject whilst reducing a chance that important information will be overlooked.

### SUMMARY OF THE INVENTION

The invention is defined by the claims.

According to examples in accordance with an aspect of the invention, there is provided a computer-implemented method for providing assistive information for a caregiver of a subject. The computer-implemented method comprises: obtaining caregiver burden information, wherein the caregiver burden information indicates a current workload of the caregiver; obtaining information quantity data indicating the amount of information of the subject currently available to the caregiver at a first user interface for the subject; processing the caregiver burden information and the information quantity data to determine a recommended level of assistance to provide to the caregiver; and controlling, responsive to the recommended level of assistance, an assistive tool configured to provide assistive information to the caregiver at a second user interface.

The present disclosure relates to approaches for aiding a caregiver (e.g. a clinician) in assessing or understanding the current condition of a subject (e.g. a patient). An assistive tool is configured to change its operation responsive to a determined recommended level of assistance. The recommended level of assistance is determined from caregiver burden information (which indicates a cognitive load or burden of the caregiver) and information quantity data (which represents an amount of data available to the caregiver). These two quantities of information affect an ability of the caregiver to comprehend and take account of all relevant information for accurately assessing the condition of the subject (due to mental or visual/audio overload).

By way of further explanation, it is recognized that a caregiver overloaded with information becomes desensitized, with decreased alertness and confidence to the urgency or the importance of critical information available at a user interface.

Controlling the assistive tool based on the recommended level of assistance thereby provides a mechanism by which additional assistance can be provided when the caregiver is predicted to be overwhelmed, and reduce the additional assistance if the caregiver is able to perform assessment correctly.

The proposed invention solves the above problems with an approach that modifies the functionality of an assistive tool based on the caregiver burden information and information quantity data This may comprise deciding whether or not to provide additional information for aiding the caregiver or to emphasize critical values or medical information of the subject.

The assistive tool may be configured to respond to a user input, containing an information request, at the second user interface with information about the subject pertaining to the information request; and the step of controlling the assistive tool comprises controlling the amount and/or content of the information provided by the assistive tool in response to the information request.

The assistive tool may be configured to operate in at least two different modes. The at least two different modes may comprise at least: a first mode in which the assistive tool provides only the information requested in the information request; and a second mode in which the assistive tool provides the information requested in the information request and additional information, the additional information comprising information for contextualizing the requested information and/or supplementing the requested information.

The step of determining a recommended level of assistance may comprise selecting one of the (different) modes; and the step of controlling the assistive tool may comprises controlling the assistive tool to operate in the selected mode.

The additional information preferably contains medically relevant information to the requested information. Medically relevant information is any information that may influence a medical or clinical decision made on requested information. This reduces a likelihood that medically important information will be overlooked when a caregiver is overworked or there is a large quantity of subject information to review.

By way of example, if the cardiac output (CO) of the subject is below some predetermined threshold, and the caregiver requests an arterial pressure (ATP) reading via the user input, the assistive tool may be configured to (in addition to providing the ATP reading), also provide (as additional information) information on CO being a potential parameter to watch out for.

The assistive tool may be configured to: operate in the first mode responsive to the caregiver burden information indicating that the current workload on the caregiver does not breach a first predetermined threshold and/or the amount of information currently available to the subject at the first user interface does not breach a second predetermined threshold; and operate in the second mode responsive to the caregiver burden information indicating that the current workload on the caregiver breaches the first predetermined threshold and/or the amount of information currently available to the subject at the first user interface breaches the second predetermined threshold.

The second mode may comprise at least a first sub-mode and a second sub-mode, wherein: when the assistive tool operates in the first sub-mode, the assistive tool provides additional information to supplement the requested information; and when the assistive tool operates in the second sub-mode, the assistive tool provides guidance information for emphasizing information currently available to the subject.

In some examples, the assistive tool operates in the first sub-mode responsive to the caregiver burden information indicating that the current workload on the caregiver breaches the first predetermined threshold and the amount of information currently available to the subject at the first user interface does not breach the second predetermined threshold; and the assistive tool operates in the second sub-mode responsive to the caregiver burden information indicating that the current workload on the caregiver does not breach the first predetermined threshold and the amount of information currently available to the subject at the first user interface breaches the second predetermined threshold.

In some examples, the second mode further comprises a third sub-mode in which, when the assistive tool operates in the third sub-mode, the assistive tool provides additional information to supplement the requested information and guidance information for emphasizing information currently available to the subject

In some examples, the assistive tool operates in the third sub-mode responsive to the caregiver burden information indicating that the current workload on the caregiver breaches the first predetermined threshold and the amount of information currently available to the subject at the first user interface breaches the second predetermined threshold.

The step of obtaining the caregiver burden information may comprise: obtaining burden affecting information comprising: subject information indicating a number, medical condition and/or complexity of one or more medical subjects under the responsibility of the caregiver; expertise information indicating an experience and/or training level of the caregiver; time information indicating a time at which the caregiver is giving care to one or more medical subjects; and/or activity information indicating an amount or type of activity performed by the caregiver; processing the burden affecting information to generated a predicted measure or classification of current workload of the caregiver, wherein the predicted measure or classification acts as the caregiver burden information.

A workload on a caregiver will increase for large numbers of subjects and/or complex subjects. Similarly, in a same clinical environment, a cognitive load will be higher for an inexperienced or lesser trained caregiver than a more experienced or more highly trained caregiver. Moreover, the busier a caregiver or clinical environment, the greater the cognitive load on the caregiver. Thus, a combination of one or more of these features can provide an accurate estimate or representation of the cognitive load on the caregiver.

The information quantity data comprises a measure of the amount of information available at the user interface and/or a measure of an amount of time and/or number of steps required for the caregiver to access desired information using the user interface.

In at least one example, the first user interface is a display configured to display one or more pieces of information about the subject; the assistive tool is a visual emphasization tool configured to visually emphasize one or more pieces of information displayed by the first user interface; and the step of controlling the assistive tool comprises controlling which or how many pieces of information are visually emphasized by the assistive tool.

The first and second user interface may be the same. Of course, in other embodiments, the first and second user interfaces may be distinct from one another (e.g. the first user interface may be located proximate to the subject and the second user interface may be located remotely).

There is also proposed a computer program product comprising computer program code means which, when executed on a computing device having a processing system, cause the processing system to perform all of the steps of any herein described method.

There is also proposed a processing system configured to provide assistive information for a caregiver of a subject. The processing system is configured to: obtain caregiver burden information, wherein the caregiver burden information indicates a current workload on the caregiver; obtain information quantity data indicating the amount of information of the subject currently available to the caregiver at a first user interface for the subject; process the caregiver burden information and the information quantity data to determine a recommended level of assistance to provide to the caregiver; and control, responsive to the recommended level of assistance, an assistive tool configured to provide assistive information to the caregiver at a second user interface.

The processing system may be configured to perform any herein described method, and vice versa.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates a system in which embodiments can be implemented;
Figure 2 illustrates a method according to an embodiment; and
Figure 3 illustrates a processing system according to an embodiment,

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for controlling how assistance is provided to a caregiver or clinician. Information about a workload and amount of information available to the caregiver is obtained. This information is processed to determine a recommended level of assistance that should be provided to the caregiver. The operation of an assistance tool, which may be run by a processing system, is modified based upon the recommended level of assistance.

Embodiments are based upon the realization that there is an increased likelihood of a caregiver overlooking potentially important clinical information when they are under a high workload stress and/or there is a large amount of data of a subject to review. This is contrasted with a desire to avoid continually providing caregivers with excessive assistance, to reduce a chance of annoying the caregiver or causing bias in the caregiver (e.g. authority or framing bias). By modifying a level of assistance based on workload and/or amount of information available, both of these concerns can be overcome.

The proposed concepts can be employed in any clinical environment, such as a hospital or care home, in which information of a subject is to be provided to a clinician. Embodiments are particularly advantageous in intensive care units, as these environments may provide a large amount of monitoring data for a caregiver to assess and interpret, as well as being high-stress environments.

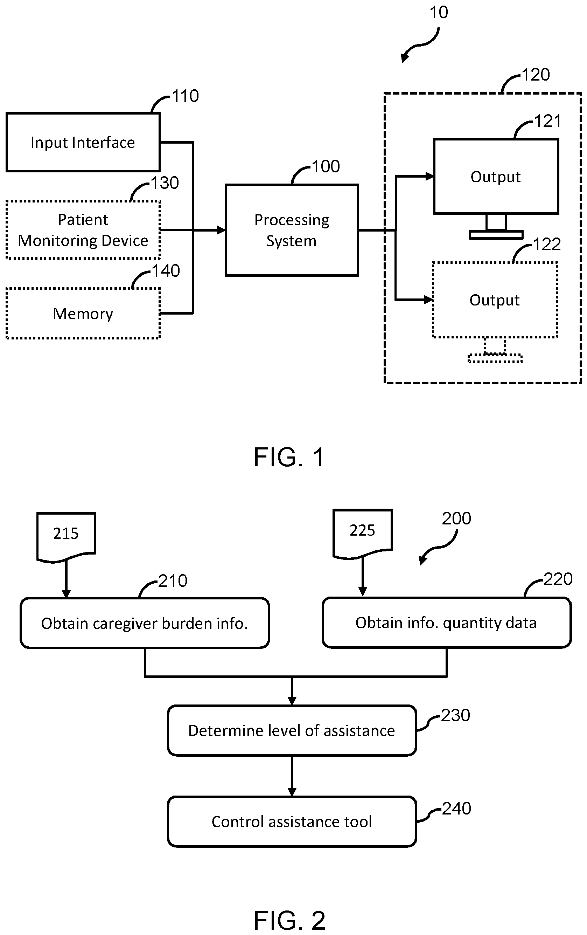

Figure 1 illustrates a system 10 in which embodiments can be implemented. The system 10 comprises a processing system 100. The system 10 may provide a patient monitor for providing information about a patient or subject.

The processing system is configured to control a user output interface 110 and receive input from a user input interface 120. The processing system 100 is also configured to receive and process data received from a subject monitoring device 130 and/or a memory 140 (which form optional components of the system 10).

The processing system 100 is configured to provide assistive information at the user output interface 110 for aiding a caregiver in performing a clinical task, such as diagnosing, assessing and/or reviewing a subject.

For instance, the processing system may provide current values of one or more physiological parameters of a subject (based on sensor signal data provided by the subject monitoring device 130 monitoring the subject) or provide information on a medical history of a subject (e.g. as provided by the memory 140). Various other forms of assistive information would be readily apparent to the skilled person.

The processing system 100 may thereby provide an assistive tool for aiding a caregiver in the performance of their tasks, duties or responsibilities.

The user output interface 110 is configured to provide one or more user-perceptible outputs (e.g. a visual, audio and/or haptic output) responsive to communications from the processing system. Thus, the processing system may provide assistive information in the form of a user-perceptible output provided at the user output interface 110.

The processing system may provide information of the subject at a first user interface 121, and provide assistance (information) at a second user interface 122. The first and second user interface may be the same or different (as illustrated).

Optionally, the operation of the (assistive tool provided by the) processing system 100 may be responsive to a user input provided at the user input interface 120. In particular, the processing system may respond to a request contained in a user input (provided at the user input interface) to provide information responsive to the request. By way of example, a user may request information on a family history of a subject, and the processing system 100 may respond to the request by retrieving the family history from the memory 140 and providing a visual representation of the family history at the user output interface 110.

The information responsive to the request may be provided at a second user interface of the user output interface 110. The second user interface may be the same as the first user interface (e.g. at which information on the subject is already present) or a different user interface.

Thus, in one example, the processing system may provide information on the subject at a first user interface (e.g. real-time values) and provide information responsive to a request from the caregiver (via the user input interface) at a second user interface. The first and the second user interface may be the same (e.g. a single display) or may be different (e.g. the first user interface may be a display and the second user interface may be an audio output).

This provides an example of how information on a subject may be provided at a first user interface and assistance may be provided (by the assistive tool) at a second user interface.

The assistive tool provided by the processing system may respond to the request in real-time (i.e. immediately after receiving the request) and/or after some period of time has elapsed and/or some predetermined trigger has been met. The period of time and/or trigger may be defined by the request, e.g. the request may indicate "alert 30 min before subject is predicted to suffer from shock", or "give a subject status update every 30 min".

The information provided by the processing system at the user interface may therefore be manipulated by a caregiver using the user input interface 120.

In such examples, the assistive tool may, for instance, take the form of a chatbot configured to automatically respond to a user request (e.g. in the form of textual or audio input) with information about the subject (e.g. in textual/audio output).

One example of a suitable assistive tool is described by the International Patent Applications having publication no. WO 2020/249718 A1 or WO 2010/036858 A1, which responds to user requests with information about a subject. Other examples include those presented in US patent application no. US 2003/033151 A1 and US patent US 6,785,358 B2.

Another form of assistive tool may be a gesture-based responder, in which the input to the processing system comprises image/video data of a gesture of the subject, wherein the processing system automatically identifies the gesture and provides information responsive to the gesture (e.g. a pat on the caregiver's chest may be a gesture to provide heartrate information).

One suitable example of a gesture-based device is disclosed by the International Patent Application having publication no. WO 2013/168056 A1 or European patent application having publication no. EP 2,793,704 A1.

Other forms and adaptations for assistive tools responsive to a user input will be apparent to the skilled person (e.g. tools that provide a model/avatar of the subject that can be manipulated by the caregiver and so on). One suitable example is provided by the European Patent EP2769270.

However, it is not essential that an assistive tool (provided by the processing system) be responsive to a user input.

For instance, another form of assistive tool may detect when a physiological parameter of the subject breaches a threshold, and generate an alarm in response to the breach. Yet another form of assistive tool may detect when a combination of physiological parameters indicates a likely clinical deterioration of the subject, and present information for avoiding the probably clinical deterioration.

Yet another assistive tool may simply provide (monitored) information about the subject at the user interface.

Embodiments of the present disclosure propose approaches for modifying the operation of the assistive tool, i.e. of the aid provided by the processing system 100, based upon a workload of a caregiver and the amount of information available (e.g. at a user interface) to the caregiver. Embodiments recognize that there is an increased likelihood that important clinical information will be overlooked by a busy or overworked caregiver, especially if there is already a substantial amount of information available for their review. By controlling the operation of the assistive tool, potentially overlooked information can be identified and emphasized and/or provided to the subject.

In some examples, modifying the operation of the assistive tool may comprise modifying an amount and/or type of information provided by the assistive tool responsive to a user input. In this way, the assistive tool can more effectively assist the based on their current workload and information available to the caregiver.

Figure 2 illustrates a method 200 for performing an embodiment of the invention. The method 100 may be performed by a processing system, such as the processing 100 described with reference to Figure 1.

The method 200 comprises a step 210 of obtaining caregiver burden information 215. The caregiver burden information indicates a current workload (i.e. burden) of the caregiver. A workload represents a cognitive and/or physical availability of the subject, e.g. representing a level of burnout, fatigue, time availability and/or other similar factors.

In the following working example, the caregiver burden information indicates whether a workload of the caregiver is "high" or "low", i.e. forms a binary indicator of workload. Put another way, in this example, the caregiver burden information may classify a workload of the caregiver as either "high" or "low". An indicator of "high" indicates that the current workload on the caregiver breaches a first predetermined threshold, whereas indicator of "low", indicates that the current workload on the caregiver does not breach a first predetermined threshold.

However, in other examples, the caregiver burden information is a numeric indicator of workload (e.g. on a predetermined scale, e.g. a scale of 0 to 1, 0 to 10 or 0 to 100) or a (multi-level, i.e. more than 3 levels) categorical indicator of workload (e.g. indicating "high", "medium" or "low").

The caregiver burden information may be generated and provided by any suitable system that monitors information about the caregiver or subject (under the responsibility of the caregiver) and/or stores information about the caregiver or subject. In some instances, step 210 may itself comprise generating the caregiver burden information.

The load on the caregiver can be estimated (i.e. the caregiver burden information generated) by processing data from multiple sources, e.g. such as from the electronic medical record (EMR) of the subject, by monitoring activity of the caregiver and/or from data of the caregiver.

Thus, step 210 may comprise obtaining burden affecting information and processing the burden affecting information to generate a predicted measure or classification of current workload of the caregiver, wherein the predicted measure or classification acts as the caregiver burden information.

The burden affecting information may comprise subject information indicating a medical condition and/or complexity of one or more medical subjects under the responsibility of the caregiver; time information indicating a time at which the caregiver is giving care to one or more medical subjects; expertise information indicating an experience and/or training level of the caregiver; values of physiological characteristics of the clinician and/or activity information indicating an amount or type of activity performed by the caregiver. The physiological characteristics may be those that respond to a stress, workload, cognitive responsiveness or fatigue of the physician.

By way of example, the caregiver burden information may be responsive to a number of subjects under the responsibility of the caregiver. For instance, if the number of subjects exceeds a first threshold subject level, then the caregiver burden information may indicate that the workload of the caregiver is "high", otherwise, it may be indicated as "low".

As another simple example, the caregiver burden information may be responsive to a time at which the caregiver is responsible for the subject(s). A workload on a caregiver is typically greater during the day than during the night (e.g. as daytime has been identified as increasing a likelihood of issues and/or other responsibilities for a caregiver. Thus, in one example, a workload may be indicated as being "low" during the night and "high" during the day.

In some examples, the caregiver burden information may be (further) responsive to a complexity or risk of the subject(s) under the responsibility of the caregiver, as the more (medically) complex a subject, or the greater the potential risk, the greater the burden on (or workload of) the caregiver responsible for the subject.

Identification of the subjects under the responsibility of the caregiver may be derived, for instance, from a duty roster (indicating currently responsibly caregivers) and/or image/video data (which may indicate which caregivers are attending which subjects). Other approaches will be apparent to the skilled person, e.g. by tracking which caregivers interact with which subjects, e.g. based on proximity sensors or log-in details.

By way of a simple example, a measure of the amount of data stored in the EMR for the subject (e.g. the number of fields containing information and/or the total data size of the EMR) may represent the complexity/risk of the subject and thereby the load on the caregiver.

As another example, more granular information from the EMR may be processed to estimate a complexity of the subject in the generation of the caregiver burden information. For instance, a number of medications taken by the subject may reflect a complexity of the subject (due to potential drug-drug interactions). In other examples, conditions of the subject and/or lab test results may indicate a complexity or risk of the subject. A combination of these features may be used and processed to assess a complexity or risk of the subject.

The caregiver burden information may, for instance, indicate that the workload on the caregiver is "high" when the caregiver is required to attend two or more high risk patients or three or more normal risk patients, and "low" otherwise. Effectively, this means that the threshold subject level may change responsive to a complexity or risk of the subject(s).

Information of a caregiver (e.g. their role, experience level, training level or responsibilities) can be used in the prediction or estimation of the caregiver workload. Identification of the current caregiver may be derived, for instance, from a duty roster (indicating currently responsibly caregivers).

Background information of the caregiver (e.g. their experience or training level) may provide further information for defining their load. By way of explanation, it can be assumed that an inexperienced caregiver will face a higher workload compared to an experienced caregiver when performing a same task. The workload could also be considered "high" when the caregiver is inexperienced and is required to attend a high risk patient. Effectively, the threshold subject level may therefore change responsive to the experience/training level of the caregiver.

Some other approaches for estimating a load can be performed by monitoring (past or current) activity, actions or information of the caregiver. Information on the (activity or actions of the) caregiver may be derived from image data and/or stored information about the caregiver.

For instance, image/video data of the caregiver may be monitored and processed to estimate the load on the caregiver. It is recognized that actions of the caregiver that can be monitored via a camera (e.g. an urgency or speed of caregiver movements and/or amount of (non-)medical related tasks being performed) are indicative of a load on caregiver. The more active a caregiver and/or the more medical tasks being performed, the greater their workload.

Thus, image/video data of the caregiver can be processed, e.g. using a machine-learning method and/or image recognition algorithm, in order to estimate a load on the caregiver.

As another example, historic data of the actions of a caregiver may be used to assess a workload of the caregiver. For instance, information about caregiver activities in a last hour (or other predefined time period) may be used to assess a workload of the caregiver. By way of example, if the caregiver has performed fewer than a threshold number of tasks in the predetermined time period, then their workload may be "low", whereas greater than this number may indicate a "high" workload. As another example, if a caregiver has been involved in intensive activity (e.g. requiring a high level of clinical input) for the predetermined time period, then their workload may be "high", otherwise it may be "low".

The foregoing examples clearly demonstrate a number of approaches as to how information about the caregiver and/or subjects under the responsibility of the caregiver can be processed to assess a workload of the caregiver. A combination of any of the above described approaches could be used. For instance, a combination of the foregoing factors could be used to determine the caregiver burden information.

Other approaches for generating caregiver burden information include methods for estimating clinician workload, such as those set out in by: Griffiths, Peter, et al. "Nursing workload, nurse staffing methodologies and tools: A systematic scoping review and discussion." International Journal of Nursing Studies 103 (2020): 103487; Margadant, Charlotte C., et al. "Nurse Operation Workload (NOW), a new nursing workload model for intensive care units based on time measurements: An observational study." International Journal of Nursing Studies 113 (2021): 103780; or Henney, C. R., et al. "A method of estimating nursing workload." Journal of advanced nursing 7.4 (1982): 319-325.

Yet another approach may be to use a machine-learning method, such as a neural network, to process information on the clinician and/or the subjects under the responsibility of the clinician to predict a workload of the clinician. The information on the clinician and/or the subject may include, amongst other possibilities, patient data, workflow information, time of day, expertise, location information, clinician movement information and so on etc. Further examples include speech (or speech features), heart rate and heart rate variability, facial expressions, interaction data showing clinicians interaction with devices: such as typing related features, clicking features, eye movements and reading related features, etc.

A machine-learning algorithm is any self-training algorithm that processes input data in order to produce or predict output data. Here, the input data comprises information on a clinician and/or the subjects under the responsibility of the clinician and the output data comprises a predicted workload of the caregiver.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example values for parameters of a clinician and/or the subjects for which they are responsible. The training output data entries correspond to a measure or workload.

The training output data entries may be obtained, for instance, from annotated/labelled data provided by one or more caregivers (e.g. in the form or questionnaires asking the clinicians to evaluate their workload - where information about the caregiver and/or theie subjects is recorded and associated with the evaluation).

Another approach to assessing caregiver workload could be to determine a measure of fatigue, cognitive responsiveness, and/or physical status of the caregiver. This can be performed, for instance, by processing monitored characteristics of the caregiver (e.g. physiological parameters such as heartrate or respiratory rate, keyboard usage, eye movements, speech, facial expression, gait and so on) to derive a measure of fatigue. Examples are provided by Yamada, Yasunori, and Masatomo Kobayashi. "Detecting mental fatigue from eye-tracking data gathered while watching video." Conference on artificial intelligence in medicine in europe. Springer, Cham, 2017 or Ohnishi, Ayumi, et al. "A Method for Estimating Doctor's Fatigue Level in Operating a Surgical Robot Using Wearable Sensors." 2021 IEEE International Conference on Pervasive Computing and Communications Workshops and other Affiliated Events (PerCom Workshops). IEEE, 2021 or Fu, Rongrong, Hong Wang, and Wenbo Zhao. "Dynamic driver fatigue detection using hidden Markov model in real driving condition." Expert Systems with Applications 63 (2016): 397-411.

The method 200 also comprises a step 220 of obtaining information quantity data 225 indicating the amount of information of the subject currently available to the caregiver at a first user interface for the subject ("amount of available information"). Step 220 may comprise determining information quantity data.

In the following working example, information quantity data indicates whether an amount of available information is "high" or "low", i.e. forms a binary indicator of amount of available information. Put another way, in this example, the information quantity data may classify an amount of available information as either "high" or "low".

An indicator of "high" indicates that the amount of available information breaches a (second) predetermined threshold, whereas indicator of "low", indicates that the amount of available information does not breach the (second) predetermined threshold.

However, in other examples, the information quantity data is a numeric indicator of amount of available information (e.g. on a predetermined scale, e.g. a scale of 0 to 1, 0 to 10 or 0 to 100) or a categorical indicator of amount of available information (e.g. indicates "high", "medium" or "low").

The information quantity data may be estimated by processing a measure of the amount of information available at the first user interface and/or a measure of an amount of time and/or number of steps required for the caregiver to access desired information using the user interface.

In some examples, the measure of the amount of information may be the number of parameters (of a subject) currently available to the caregiver at the first user interface. For instance where the first user interface is a display, the measure of amount of information may comprise or be responsive to the display density of displayed information and/or the number of tabs/windows open or buried beneath other tabs at the first user interface (e.g. where each tab provides different information about the subject).

The display density may be defined as the ratio of the number of non-black pixels to the number of black pixels. For example, the information quantity data may indicate "high" if the display density is more than 60% (or some other predetermined percentage), and "low" otherwise.

The information quantity data may indicate "high" if more than a predetermined number or percentage (of all possible) number of tabs (for a display) or parameter values are provided at the first user interface, and "low" otherwise. The predetermined percentage may, for instance, be 50%, 60% or 70%. By way of example, if a first user interface is able to display up to 20 tabs/widows simultaneously, the information quantity data may indicate "high" if more than say 10 or 12 are open.

The time taken by the caregiver and the number of interactions/steps required to access a functionality or information can also be linked to information quantity data being high, as this indicates a difficulty or complexity in finding the required functionality or information.

Yet other examples of elements that could be used as or to derive information quantity data include a type and/or number of alarm settings; an amount of (available) patient data relevant to the current patient state; a display size; a type of display; a time and/or frequency at which information becomes available or is updated; the source of information (e.g. from sensors or predictive algorithms); a number of different colors in a display of information about the subject; a size of displayed numbers in a display of information about the subject and so on.

Of course, any combination of the above-described approaches could be used. For instance, information quantity data may indicate "high" if more if the display density is more than a first predetermined percentage and/or if more than a second predetermined number or percentage (of all possible) number of tabs (for a display) or parameter values are provided at the first user interface-and "low" otherwise.

Other approaches may use numeric indicators for the information quantity data, and any previously described approach may be adapted accordingly.

Tscholl, David W., et al. "It's not you, it's the design-common problems with patient monitoring reported by anesthesiologists: a mixed qualitative and quantitative study." BMC anesthesiology 19.1 (2019): 1-10 suggests a number of factors that contribute to information quantity overload for a subject, any of which could be used as a measure of information quantity data and/or used to derive information quantity data.

The method also comprises a step 230 of processing the caregiver burden information and the information quantity data to determine a recommended level of assistance to provide to the caregiver.

In particular examples, step 230 may comprise determining in which mode to operate the assistive tool. Thus, the assistive tool may be operable in a plurality of different modes, and step 230 may comprise selecting in which of these modes the assistive tool is to operate. Effectively, in this example, different modes represent different levels of assistance to provide to the caregiver.

The method 200 then performs a step 240 of controlling, responsive to the recommended level of assistance, an assistive tool configured to provide assistive information to the caregiver at a second user interface. Step 240 thereby effectively comprises controlling or adjusting the level of interaction, assistance and/or the functionality of the assistive tool based on the caregiver burden information and the information quantity data.

The first and second user interface may be the same, e.g. may comprise a same display and/or audio output device. In other examples, the first and second user interfaces are different. For instance, a first user interface may comprise a display and the second user interface may comprise an audio output device.

For instance, step 240 may comprise controlling the assistive tool to operate in the identified mode (if step 230 comprises identifying a mode).

The assistive tool may thereby operate in a number of modes, modifying its behavior and/or functionality in each mode. By way of example, different modes may represent differing amounts of (additional) information to provide to the subject responsive to a user request.

As an example, an assistive tool operating in a first mode ("mode 1") may provide only the requested information in response to a user requested. An assistive tool operating in a second mode ("mode 2) may provide the requested information and additional information for guiding the caregiver in assessing the requested information and/or supplementing the requested information.

The second mode may comprise one or more sub-modes, which provide different levels of assistance or information dependent upon the caregiver burden information and/or information quantity data.

Table 1 illustrates one approach or ruleset for defining in which mode the assistive tool operates. In this examples, the mode is selected based on a binary classification of the caregiver burden information and the information quantity data as either "high" or "low". A multi-level classification would facilitate the operation of the assistive tool in a greater number of modes if required.

**TABLE 1**

| Mode of operation | | Caregiver Burden Information | Information Quantity Data |
|---|---|---|---|
| Mode 1 | | Low | Low |
| Mode 2 | Sub-mode 1 | High | Low |
| | Sub-mode 2 | Low | High |
| | Sub-mode 3 | High | High |

Thus, the assistive tool may be made to operate in the first mode (mode 1) responsive to the caregiver burden information and information quantity data indicating "low".

In this mode, the mode 1, the assistive tool thus provides only the requested information, so that interaction with the caregiver would be at a minimum. This reduces a number of tasks performed by the assistive tool and frees up computational resource when assistance is not required (due to low stress of the caregiver).

Operation in mode 1 is preferred at low levels of workload or information overload, as consistently providing additional information could lead to cognitive biases and therefore clinical errors. For instance, consistently providing additional information can lead to: framing bias (a tendency to be swayed by subtleties in how a situation is presented (e.g., description of the risks and benefits of treatment options)), or reliance on authority bias (relying unduly on authority or technology).

When operating in the first mode, the operation of the processing system may be effectively the "default" or conventional mode of operation of the processing system.

The assistive tool made be made to operate in the second mode (mode 2) responsive to either the caregiver burden information or information quantity data. In this way, additional information is provided to the caregiver

The additional information may be in the form of supplementary information (e.g. information that goes beyond the information indicated in the request) or guidance information (e.g. information that emphasizes information indicated in the request).

The assistive tool may operate in a first sub-mode (sub-mode 1) responsive to the caregiver burden information indicating "high" and information quantity data indicating "low". In the first sub-mode the assistive tool may provide supplementary information for contextualizing or emphasizing relevant information related to (i.e. medically relevant with respect to) that indicated in the request.

For example, in a situation in which the cardiac output (CO) of the subject is borderline low (i.e. has breached a particular threshold), if the caregiver asks the assistive tool to read out arterial pressure (ATP), in addition to providing this info, the assistive tool may (when operating in the first sub-mode) also provides information on CO being a potential parameter to watch out for.

As another example, the assistive tool may be configured to (when operating in the first sub-mode) suggest the most likely diagnosis or crucial procedure. This helps or aids a caregiver in avoiding tunnel vision or problem fixation related errors due to being overworked, and helps suggest other routes for the caregiver to investigate.

As yet another example, the assistive tool may be configured to, when operating, in the first sub-mode, modify the presentation of information at the first user interface. For instance, if the first user interface provides visual representations of different parameters of the subject (e.g. in different tabs), then the visual representations may be ordered based on a proximity of the respective parameter to an abnormal range and/or alarm thresholds.

The assistive tool may operate in a second sub-mode (sub-mode 2) responsive to the caregiver burden information indicating "low" and information quantity data indicating "high".

Effectively, in the second sub-mode, the load on the caregiver is low, but the information crowding the first user interface is high. This makes it harder for a caregiver find the required information, and increases the likelihood of missing crucial info due to oversight.

To overcome these issues, when operating in the second sub-mode the assistive tool may emphasize information (e.g. that might otherwise be missed due to overcrowding of information).

For instance, the assistive tool may emphasize crucial information both audibly and visually, along with responding to the requested information. In this way, the assistive tool helps reduce errors by ensuring that relevant information is provided at the right time to the caregiver.

As another example, where the second user interface is a display, a window may pop-up with all the parameters (of the subject) that have breached thresholds and/or have triggered an alarm. In this way, the window in the second user interface will only contain parameters which are deemed "crucial" (as they have breached the threshold). Based on the severity of the breach, those parameters can be additionally emphasized both audibly and visually.

The assistive tool may operate in a third sub-mode (sub-mode 3) responsive to the caregiver burden information indicating "high " and information quantity data indicating "high". In other words, both the load on the caregiver and the quantity of information available to the caregiver is high

In the third sub-mode, the functionalities of the first and second sub-modes are provided at the same time.

For instance, if the first user interface provides visual representations of different parameters of the subject (e.g. in different tabs), the tabs are rearranged as set out in the example for the description of the first sub-mode. In addition, a pop up screen is layered on top with all parameters deemed crucial as set out in the example for the description of the second sub-mode.

The various modes and sub-modes thereby provide additional aid or assistance to the caregiver if they are overworked and/or if there is a large quantity of medical data for their review, without providing this information if they are not overworked (or there is a low quantity of medical data for review) to reduce the likelihood of over-reliance on automated recommendations and reduce processing power.

In some circumstances, it may not be possible for either the caregiver burden information or information amount data may to be calculated (e.g. due to missing information or an error). Steps 230 and 240 may be configured to continue operation in these circumstances by either assuming that the missing parameter does not breach the relevant threshold or (temporarily) replacing the missing parameter by the median (or other average) of the data collected during a time period before the parameter went missing, e.g. during a current shift of the caregiver or for a previous predetermined period of time. This may be in place until the parameter becomes available again.

Other approaches for selecting a mode of the assistive tool may be employed, e.g. if the caregiver burden information and information quantity data provide non-binary indicators.

For instance, in some embodiments the caregiver burden information and information quantity data each provide a numeric indicator. The numeric indicators may be processed together to calculate a measure or single indicator (e.g. a ratio may be determined), and this measure or single indicator may be compared to one or more thresholds.

Thus, more generally, a mode operation decision may be made based upon the output of a predefined function that receives, as input, the caregiver burden information and the information quantity data.

Previous examples for steps 230 and 240 describe modifications to perform to an assistive tool when the assistive tool is configured to response to a user request with information requested in the user request, and specifically relate to modifying a mode of the assistive tool which defines how the assistive tool responds to the user request.

However, other forms of assistive tools could be modified according to embodiments of the invention.

For instance, one assistive tool may respond to a request for guidance through performing a particular medical procedure (e.g. cardiogenic shock case). In one example, if the caregiver burden information indicates "low" and (optionally) the information quantity data indicates "high", then only the crucial steps of the medical procedure may be provided, e.g. to reduce the likelihood of further overcrowding of information. However, if the caregiver burden indicates "high" (e.g. regardless of the information quantity data) then each step of the medical procedure may be provided, e.g. to reduce the likelihood of an overworked caregiver missing a step.

In another scenario, one form of assistive tool may provide a caregiver perceptible alert responsive to a monitored parameter of the subject breaching some predetermined threshold. In one example, the information contained in the alert may be modified based on the recommended level of assistance (i.e. based on the caregiver burden information and the information quantity data).

In this scenario, the assistive tool may be configured to provide a simple alert to a caregiver if both caregiver burden information and the information quantity data indicate "low". In one example, the assistive tool may provide an alert and a predicted cause of the alert if either the caregiver burden information or the information quantity data is "high". In an example, the assistive tool may (visually) emphasize a provision of the monitored parameter (that breached the threshold) at the first user interface if the information quantity data indicates "high".

In yet another scenario, the assistive tool may be a visual/audio emphasization tool configured to visually/audibly emphasize one or more pieces of information displayed or provided by a user interface. Controlling the assistive tool may comprise controlling which or how many pieces of information are visually/audibly emphasized by the assistive tool. For instance, no pieces of information may be visually/audibly emphasized if both caregiver burden information and the information quantity data indicate "low" and one or more pieces of information may be visually/audibly emphasized if either caregiver burden information and the information quantity data indicate "low". The visually/audibly emphasized piece(s) of information may, for instance, be responsive to a condition or diagnosis of the subject (e.g. cardiac patients may have information about the heart emphasized).

The above description provides only a few examples of potential assistive tools and mechanisms for modifying such assistive tools. The skilled person would readily appreciate a number of other assistive tools and ways to modify an assistive tool following the teaching of the present disclosure that such modifications may be made responsive to caregiver burden information and/or information quantity data.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

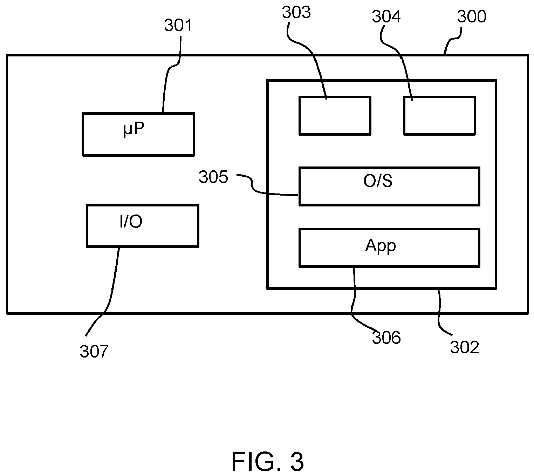

By way of further example, Figure 3 illustrates an example of a computer 300 within which one or more parts of an embodiment may be employed. Various operations discussed above may utilize the capabilities of the computer 300. For example, one or more parts of a system for providing assistance information to a caregiver may be incorporated in any element, module, application, and/or component discussed herein. In this regard, it is to be understood that system functional blocks can run on a single computer or may be distributed over several computers and locations (e.g. connected via internet).

The computer 300 includes, but is not limited to, PCs, workstations, laptops, PDAs, palm devices, servers, storages, and the like. Generally, in terms of hardware architecture, the computer 300 may include one or more processors 301, memory 302, and one or more I/O devices 307 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 301 is a hardware device for executing software that can be stored in the memory 302. The processor 301 can be virtually any custom made or commercially available processor, a central processing unit (CPU), a digital signal processor (DSP), or an auxiliary processor among several processors associated with the computer 300, and the processor 301 may be a semiconductor based microprocessor (in the form of a microchip) or a microprocessor.

The memory 302 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 302 may incorporate electronic, magnetic, optical, and/or other types of storage media. Note that the memory 302 can have a distributed architecture, where various components are situated remote from one another, but can be accessed by the processor 301.

The software in the memory 302 may include one or more separate programs, each of which comprises an ordered listing of executable instructions for implementing logical functions. The software in the memory 302 includes a suitable operating system (O/S) 305, compiler 304, source code 303, and one or more applications 306 in accordance with exemplary embodiments. As illustrated, the application 306 comprises numerous functional components for implementing the features and operations of the exemplary embodiments. The application 306 of the computer 300 may represent various applications, computational units, logic, functional units, processes, operations, virtual entities, and/or modules in accordance with exemplary embodiments, but the application 306 is not meant to be a limitation.

The operating system 305 controls the execution of other computer programs, and provides scheduling, input-output control, file and data management, memory management, and communication control and related services. It is contemplated by the inventors that the application 306 for implementing exemplary embodiments may be applicable on all commercially available operating systems.

Application 306 may be a source program, executable program (object code), script, or any other entity comprising a set of instructions to be performed. When a source program, then the program is usually translated via a compiler (such as the compiler 304), assembler, interpreter, or the like, which may or may not be included within the memory 302, so as to operate properly in connection with the O/S 305. Furthermore, the application 306 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 307 may include input devices such as, for example but not limited to, a mouse, keyboard, scanner, microphone, camera, etc. Furthermore, the I/O devices 307 may also include output devices, for example but not limited to a printer, display, etc. Finally, the I/O devices 307 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 307 also include components for communicating over various networks, such as the Internet or intranet.

If the computer 300 is a PC, workstation, intelligent device or the like, the software in the memory 302 may further include a basic input output system (BIOS) (omitted for simplicity). The BIOS is a set of essential software routines that initialize and test hardware at startup, start the O/S 305, and support the transfer of data among the hardware devices. The BIOS is stored in some type of read-only-memory, such as ROM, PROM, EPROM, EEPROM or the like, so that the BIOS can be executed when the computer 300 is activated.

When the computer 300 is in operation, the processor 301 is configured to execute software stored within the memory 302, to communicate data to and from the memory 302, and to generally control operations of the computer 300 pursuant to the software. The application 306 and the O/S 305 are read, in whole or in part, by the processor 301, perhaps buffered within the processor 301, and then executed.

When the application 306 is implemented in software it should be noted that the application 306 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 306 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

Ordinal numbers (e.g. "first", "second" and so on) have been used purely to distinguish different elements from one another for the sake of clarity, and reference to a non-"first" (e.g. "second" or "third") element does not necessitate that a "first" element be present. The skilled person would be capable of relabeling any such elements as appropriate (e.g. relabeling a "second" element as a "first" element if only the second element is present).

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A computer-implemented method (200) for providing assistive information for a caregiver of a subject, the computer-implemented method comprising:
obtaining (210) caregiver burden information (215), wherein the caregiver burden information indicates a current workload of the caregiver;
obtaining (220) information quantity data (225) indicating the amount of information of the subject currently available to the caregiver at a first user interface (121) for the subject;
processing (230) the caregiver burden information and the information quantity data to determine a recommended level of assistance to provide to the caregiver; and
controlling (240), responsive to the recommended level of assistance, an assistive tool configured to provide assistive information to the caregiver at a second user interface (122).

2. The computer-implemented method of claim 1, wherein:
the assistive tool is configured to respond to a user input, containing an information request, at the second user interface with information about the subject pertaining to the information request; and
the step of controlling the assistive tool comprises controlling the amount and/or content of the information provided by the assistive tool in response to the information request.

3. The computer-implemented method of claim 2, wherein:
the assistive tool is configured to operate in at least two different modes;
the step of determining a recommended level of assistance comprises selecting one of the modes; and
the step of controlling the assistive tool comprises controlling the assistive tool to operate in the selected modes, wherein the at least two different modes includes:
a first mode in which the assistive tool provides only the information requested in the information request; and
a second mode in which the assistive tool provides the information requested in the information request and additional information, the additional information comprising information for contextualizing the requested information and/or supplementing the requested information.

4. The computer-implemented method of claim 3, wherein the additional information contains medically relevant information to the requested information.

5. The computer-implemented method of any of claims 3 to 4, wherein the assistive tool is configured to:
operate in the first mode responsive to the caregiver burden information indicating that the current workload on the caregiver does not breach a first predetermined threshold and/or the amount of information currently available to the subject at the first user interface does not breach a second predetermined threshold; and
operate in the second mode responsive to the caregiver burden information indicating that the current workload on the caregiver breaches the first predetermined threshold and/or the amount of information currently available to the subject at the first user interface breaches the second predetermined threshold.

6. The computer-implemented method of any of claims 3 to 5, wherein the second mode comprises at least a first sub-mode and a second sub-mode, wherein:
when the assistive tool operates in the first sub-mode, the assistive tool provides additional information to supplement the requested information; and
when the assistive tool operates in the second sub-mode, the assistive tool provides guidance information for emphasizing information currently available to the subject.

7. The computer-implemented method of claim 6, when dependent upon claim 5, wherein:
the assistive tool operates in the first sub-mode responsive to the caregiver burden information indicating that the current workload on the caregiver breaches the first predetermined threshold and the amount of information currently available to the subject at the first user interface does not breach the second predetermined threshold; and
the assistive tool operates in the second sub-mode responsive to the caregiver burden information indicating that the current workload on the caregiver does not breach the first predetermined threshold and the amount of information currently available to the subject at the first user interface breaches the second predetermined threshold.

8. The computer-implemented method of claim 6 or 7, wherein the second mode further comprises a third sub-mode in which, when the assistive tool operates in the third sub-mode, the assistive tool provides additional information to supplement the requested information and guidance information for emphasizing information currently available to the subject

9. The computer-implemented method of claim 8, when dependent upon claim 5, wherein the assistive tool operates in the third sub-mode responsive to the caregiver burden information indicating that the current workload on the caregiver breaches the first predetermined threshold and the amount of information currently available to the subject at the first user interface breaches the second predetermined threshold.

10. The computer-implemented method of any of claims 1 to 9, wherein the step of obtaining the caregiver burden information comprises:
obtaining burden affecting information comprising:
subject information indicating a number, medical condition and/or complexity of one or more medical subjects under the responsibility of the caregiver;
expertise information indicating an experience and/or training level of the caregiver;
time information indicating a time at which the caregiver is giving care to one or more medical subjects; and/or
activity information indicating an amount or type of activity performed by the caregiver;
processing the burden affecting information to generated a predicted measure or classification of current workload of the caregiver, wherein the predicted measure or classification acts as the caregiver burden information.

11. The computer-implemented method of any of claims 1 to 10, wherein the information quantity data comprises a measure of the amount of information available at the user interface and/or a measure of an amount of time and/or number of steps required for the caregiver to access desired information using the user interface.

12. The computer-implemented method of any of claims 1 to 11, wherein:
the first user interface is a display configured to display one or more pieces of information about the subject;
the assistive tool is a visual emphasization tool configured to visually emphasize one or more pieces of information displayed by the first user interface; and
the step of controlling the assistive tool comprises controlling which or how many pieces of information are visually emphasized by the assistive tool.

13. The computer-implemented method of any of claims 1 to 12, wherein the first and second user interface are the same.

14. A computer program product comprising computer program code means which, when executed on a computing device having a processing system, cause the processing system to perform all of the steps of the method according to any of claims 1 to 13.

15. A processing system (300) configured to provide assistive information for a caregiver of a subject, the processing system being configured to:
obtain (210) caregiver burden information (215), wherein the caregiver burden information indicates a current workload on the caregiver;
obtain (220) information quantity data (225) indicating the amount of information of the subject currently available to the caregiver at a first user interface for the subject;
process (230) the caregiver burden information and the information quantity data to determine a recommended level of assistance to provide to the caregiver; and
control (240), responsive to the recommended level of assistance, an assistive tool configured to provide assistive information to the caregiver at a second user interface.
